# FASCICULE DE BREVET EUROPEEN

(11) **EP 3 376 217 B1**
(45) Date de publication et mention de la délivrance du brevet: **18.12.2019**
(21) Numéro de dépôt: 17160721.1
(22) Date de dépôt: 14.03.2017
(51) Int. Cl.: G01N 29/02, A61M 1/36, A61M 5/36, G01N 29/22, G01N 29/24, G01N 29/032

(54) **ENSEMBLE POUR LA DETECTION DE GAZ CONTENU DANS UN LIQUIDE**
EINHEIT ZUR DETEKTION VON GAS, DAS IN EINER FLÜSSIGKEIT ENTHALTEN IST
ASSEMBLY FOR DETECTING GAS CONTAINED IN A LIQUID

(43) Date de publication de la demande: 19.09.2018
(73) Titulaire: Infomed SA, 1227 Acacias (CH)
(72) Inventeur: ZALUNARDO, Ivano, 1219 Châtelaine (CH)
(74) Mandataire: Micheli & Cie SA

(56) Documents cités:
- WO-A1-2015/191775
- US-A- 5 179 862
- US-B2- 8 986 252

## Description

La présente invention concerne un dispositif qui permet de détecter la présence de gaz dans un liquide confiné ou circulant dans une conduite.

De nombreuses situations requièrent d'identifier la présence de gaz, souvent de l'air, dans un liquide confiné ou circulant dans une conduite. Dans le domaine médical par exemple, il s'agit fréquemment de détecter la présence d'air, dont l'injection peut s'avérer dangereuse pour le patient, dans une conduite contenant un liquide que l'on injecte à un patient, par exemple du sang ou un médicament.

Il en est de même dans les systèmes de circulation de fluides utilisés dans le domaine de l'épuration extracorporelle de patients atteints par exemple de défaillances rénales et traités par des méthodes connues telles que la dialyse ou l'hémofiltration. L'invention ne se limite cependant pas à ce domaine particulier.

Pour différencier un liquide pur d'un liquide contenant un gaz, par exemple de l'air, des moyens ont été décrits par le passé qui utilisent notamment des émetteurs et récepteurs ultrasoniques. Un tel système est par exemple décrit dans le brevet US 3,921,622 qui comporte un émetteur et un récepteur ultrasoniques placés de part et d'autre de la conduite dans laquelle circule le fluide à analyser. Cette conduite est formée d'un tube légèrement écrasé entre les 2 parties comportant d'une part l'émetteur et d'autre part le récepteur. Le système décrit comporte une électronique qui alimente l'émetteur lequel génère une onde ultrasonique qui se propage à travers la conduite jusqu'au récepteur qui génère alors un courant électrique correspondant au signal reçu. Le signal est ensuite traité par l'électronique de manière à détecter l'éventuelle présence de gaz dans le liquide circulant entre les composants (émetteur et récepteur) du capteur.

Le brevet US 5,583,280 améliore la technique d'assemblage du capteur notamment en remplaçant les 2 pièces mobiles comportant l'émetteur et le récepteur par une pièce en U moulée au centre de laquelle la conduite sera placée, l'émetteur et le récepteur étant eux collés de part et d'autre des branches formant le U selon une méthode ajoutant encore à la simplification de l'assemblage.

Au besoin de détecter une éventuelle présence de gaz dans une conduite, s'ajoute souvent celui de l'utilisation aisée d'un système complexe et des contraintes de coût de production notamment pour du matériel à usage unique qui est par définition produit en grandes séries. Une solution connue à ce problème est d'intégrer le plus possible d'éléments dans une cassette. Ces éléments sont par exemple des cavités pour des pompes à membranes, des zones de clampage ou de mesure de pressions, de couleur du fluide écoulé ou de présence d'air dans un fluide injecté comme par exemple le sang du patient qui lui est retourné après son épuration. Un exemple d'une telle cassette est décrit dans le brevet US 8,033,157 qui inclus une zone rétrécie de circulation de fluide autour de laquelle un émetteur est placé en face d'un récepteur l'ensemble constituant un capteur qui permet de détecter d'éventuelles bulles d'air dans le liquide.

En général, ces cassettes sont assez complexes à fabriquer, elles possèdent plusieurs éléments, souvent des pièces moulées, qui doivent être ajustés avec précision les unes par rapport aux autres, et autant d'étapes de fabrication et d'assemblage avec à chaque fois des coûts et des risques associés, par exemple un assemblage disjoint ou des bavures résultant de l'injection de matière.

Une possibilité pour simplifier la production de telles cassettes est de concevoir une cassette réalisée avec seulement une pièce moulée, habituellement rigide et comportant des cavités, et une pièce plate obtenue par exemple par extrusion, habituellement souple et appelée par la suite « membrane », qui est fixée sur la première d'une manière quelconque (solvant, soudure ultrasonique ou laser, ...). Pour bénéficier des avantages d'un tel assemblage, il convient que la pièce moulée possède une surface plane pour fixer la membrane et ainsi fermer les conduites créées par les cavités de la pièce moulée de manière parfaitement étanche. Une telle cassette et son fonctionnement a été en partie décrite dans le brevet EP 2006543B1 au nom du demandeur. Une surface de fixation non plane de la membrane est possible mais augmente les difficultés et nécessite bien souvent que ladite membrane soit également moulée ; une telle solution n'est donc pas économique.

Pour son utilisation, une cassette telle que décrite ci-dessus est positionnée sur un appareil qui comporte des actuateurs et des capteurs. Comme pour la production de la cassette, la solution la plus simple et économique est de disposer toutes les parties de l'appareil devant interagir avec la cassette d'un même côté, limitant ainsi les positionnements relatifs en 3 dimensions et le besoin de disposer de plusieurs systèmes de positionnement des composants de l'appareil sur la cassette. Ainsi, la cassette sera de préférence placée sur un appareil qui comporte tous les actuateurs et capteurs du côté de la membrane qui est souple et permet d'interagir avec les cavités de la pièce injectée d'une manière non invasive, laquelle prévient la pollution du fluide présent dans les conduites.

Si des pompes, clamps ou capteurs de pressions à travers une membrane plane ont déjà été réalisés depuis le même côté de la cassette, dans l'art antérieur précédemment décrit la possibilité de détecter d'éventuelles bulles de gaz dans le liquide circulant dans une ou plusieurs conduite(s) d'une telle cassette, n'est réalisée qu'en plaçant un émetteur d'un côté de la cassette et un récepteur sur une face opposée. Le signal ultrasonique traverse ainsi directement la conduite à analyser. De tels systèmes ne permettent pas de placer l'ensemble des actuateurs et capteurs du même côté de la cassette, ce qui entraine des complications et des coûts puisque au moins une partie d'un capteur se retrouve sur la partie opposée de la cassette, partie qui de plus est souvent mobile pour permettre la mise en place de la cassette. Il est donc nécessaire de faire passer les câbles des capteurs à travers des éléments mobiles, ce qui augmente le risque de ruptures et empêche le montage et le contrôle dudit capteur en dehors de l'appareil monté.

Les montages électroniques reliés aux capteurs ultrasoniques sont abondamment décrits dans l'art antérieur et peuvent être appliqués sous une forme ou une autre à la présente invention.

Ainsi, dans l'art antérieur, pour détecter un gaz dans un liquide contenu ou circulant dans une conduite on place toujours l'émetteur en face du récepteur.

La publication WO 2015/191775 ainsi que le brevet US 5,179,862 décrivent un ensemble pour la détection des caractéristiques d'un liquide confié ou circulant dans une conduite, comprenant un élément de support de la conduite et un capteur permettant l'émission et la réception d'onde acoustique ou lumineuse, le capteur étant agencé sur le support de la conduite en regard d'un seul et même côté de la conduite et l'élément de support comportant un guide d'onde apte à acheminer l'onde émise par l'émetteur du capteur vers le récepteur du capteur. Le brevet US 8 986 252 décrit une cassette utilisée dans un dispositif médical de pompage pour l'administration d'un fluide médical, ladite cassette étant pourvue d'un dispositif capteur de la présence d'aie comprenant un émetteur et un récepteur disposés l'un au contact de l'autre ou séparés.

Toutefois, une configuration possédant l'émetteur et le récepteur du même côté n'est pas applicable à une cassette réalisée d'une pièce injectée sur laquelle est fixée une membrane souple, celle-ci ne permettant pas la transmission de l'énergie de l'onde incidente. Ainsi, dans les exemples qui précèdent on remarque que les capteurs par ultrasons appuient toujours sur des pièces en matériaux rigides. Ce problème est moins marqué avec un capteur optique, lequel ne permet cependant pas de distinguer facilement la présence de bulles dans un liquide très coloré comme le sang et ne peut donc être appliqué à des systèmes comme la dialyse.

Un objectif de l'invention est donc de fournir un ensemble capteur-conduite pour la détection de gaz dans un liquide confiné ou circulant dans une conduite qui est agencé de manière à minimiser l'encombrement du dispositif, en particulier dans le cas d'un liquide confiné ou circulant dans une cavité formant conduite dans une cassette de filtration fermée par une membrane souple.

Un autre objectif de l'invention est d'offrir un tel ensemble qui soit agencé du même côté que les autres actuateurs et capteurs lorsqu'il est intégré par exemple dans un système plus complexe tel qu'une cassette de dialyse. Enfin on souhaite également que cet ensemble de détection puisse être assemblé et testé en dehors de l'appareil auquel il sera intégré.

Ce but est atteint par un dispositif de détection qui se distingue par les caractéristiques énoncées à la revendication 1.

D'autres avantages ressortent des caractéristiques énoncées dans les revendications dépendantes et de la description détaillée qui suit et des figures dans lesquelles :
La figure 1 illustre à titre d'exemple et de manière schématique une forme d'exécution d'un dispositif de détection de l'état de la technique connu.
La figure 2 illustre schématiquement une forme d'exécution de l'invention lorsque la conduite est formée par une cavité d'une cassette de circulation de fluides fermée par une membrane souple.
La figure 3 est une représentation schématique d'un capteur qui comprend un émetteur et un récepteur.

Dans ce qui suit, on entend par « partie moulée » un composant réalisé de manière préférée par injection de matière plastique. Les formes permettant la réalisation de l'invention peuvent aussi être réalisées par usinage, étampage ou toute autre méthode connue de mise en forme de matière plastique ou métallique.

On entend par « conduite » une forme quelconque fermée dans laquelle un liquide peut être confiné ou circuler.

On entend par « capteur », un élément qui permet de transmettre et de recevoir une onde et qui comprend généralement un émetteur et un récepteur, les 2 éléments pouvant n'être de fait qu'un seul élément utilisé une fois en mode d'émission et une fois en mode de réception. Le capteur peut aussi recevoir plusieurs émetteurs et/ou plusieurs récepteurs.

On entend par « émetteur » un composant qui lorsqu'il est activé, par exemple par un courant électrique, génère une onde qui traverse la conduite jusqu'au récepteur. De préférence, l'onde est ultrasonique ou optique et générée par exemple par des plaquettes piézo-électriques ou des diodes luminescentes. Le « récepteur » est un élément sensible au type d'onde émise par l'émetteur qui génère un signal, par exemple un courant ou une tension électrique, correspondant au signal qu'il reçoit. Le récepteur est par exemple une plaquette piézo-électrique ou un phototransistor. Lorsque l'émetteur et le récepteur sont de fait le même composant, on appelle ce dernier « émetteur-récepteur ».

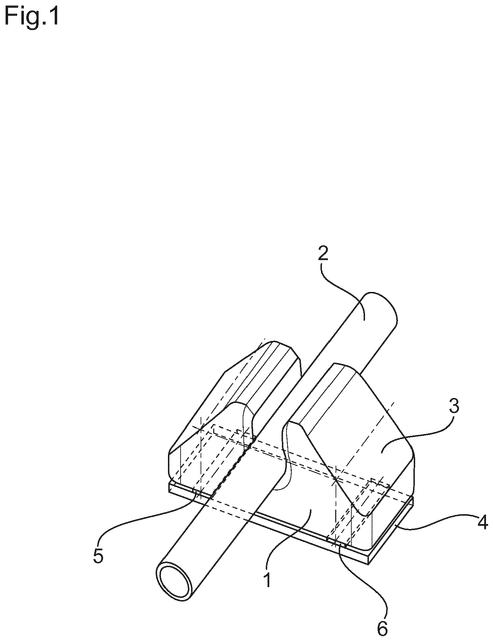

A titre d'exemple d'art antérieur connu, en référence à la figure 1, il est illustré schématiquement une conduite 2 formée d'un tube souple ou rigide dans lequel un liquide peut circuler ou être confiné. Cette conduite est agencée dans un support 1 qui présente en son centre un évidement apte à accueillir la conduite 2. Typiquement, la conduite 2 est un tube en matière plastique à usage limité qui est régulièrement remplacé alors que le support 1 est un élément fixé de manière définitive sur un appareil. Le support 1 présente en son centre deux parois entourant de chaque côté la conduite et sur ses côtés extérieurs des flancs inclinés 3, de préférence à 45 degrés, qui forment un guide d'onde pour permettre la réflexion, la transmission ou la réfraction d'une onde générée par un capteur 4 qui comprend un émetteur et un récepteur qui sera décrit plus en détail ci-après. Le capteur 4 est agencé sur le support 1, en dessous de la conduite 2. Le capteur 4 est situé d'un seul et même côté de la conduite à analyser.

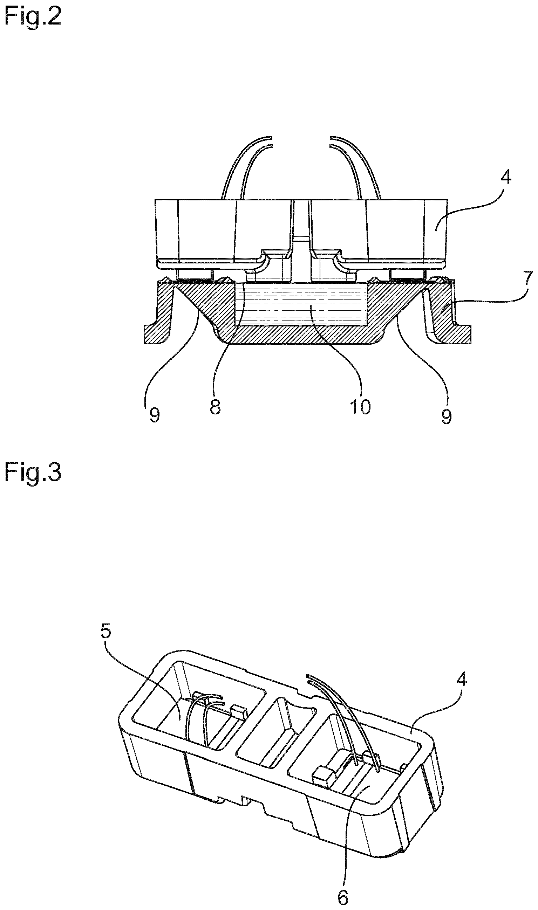

En référence à la figure 2, on a représenté en coupe un ensemble de détection de la présence d'un gaz dans un liquide selon l'invention qui comprend un capteur électronique 4 composé d'un émetteur d'onde et d'un récepteur d'onde agencés côte à côte dans le même plan. L'élément de support de la conduite est dans cette forme d'exécution préférée constitué par le corps 7 d'une cassette de circulation des fluides telle qu'une cassette de dialyse qui intègre des cavités propres à créer à travers sa membrane des pompes, des clamps, des capteurs de pression, de sang ou d'air. Ladite cassette est réalisée de préférence d'une pièce rigide moulée et d'une pièce souple extrudée.

La cassette présente un évidement 10 dans lequel le liquide peut circuler ou être confiné. Une membrane 8 est fixée par un moyen quelconque à la pièce rigide 7 formant le corps de la cassette. La membrane est fixée de façon étanche et permet d'obturer la cavité 10 pour former ainsi la conduite dans laquelle circule ou est confiné le liquide. On remarque que la membrane 8 est comprimée entre le capteur 4 et une partie rigide de la cassette, ce qui crée le couplage nécessaire à assurer la transmission de l'onde incidente au guide d'onde 9.

Cette membrane 8 est une plaque relativement fine réalisée de manière préférée dans un plastique souple par extrusion. D'autres réalisations sont possibles, par exemple en utilisant des feuilles de métal obtenues par laminage.

Le corps de la cassette formant le support de la conduite comporte en outre des guides d'ondes 9 propres à permettre le transit d'une onde entre l'émetteur 5 et le récepteur 6 du capteur 4 qui dans cette forme d'exécution est de préférence placé du même côté que la membrane souple 8.

On entend par « guides d'ondes » 3,9 des surfaces propres à transmettre, réfléchir ou réfracter les ondes émises par l'émetteur 5 vers le récepteur 6 après avoir traversé le liquide. Ces surfaces sont dans un mode préféré réalisées dans le corps de la cassette 7 par des surface agencées à 45 degrés. Ainsi comme illustré aux figures 2 et 3 l'onde est émise par l'émetteur 5 situé sur la gauche de la cassette perpendiculairement à la membrane 8 et se réfléchit horizontalement vers le flanc incliné opposé 9 sur la droite de la figure à travers le liquide. Lorsque l'onde atteint le flanc incliné 9 situé sur le côté droit de la cassette, elle est réfléchie verticalement en direction du détecteur 6. D'autres chemins de l'onde sont également possibles, par exemple en prévoyant des réflexions multiples.

Pour définir le chemin de l'onde, les lois de Snell-Descartes sont utilisées, qui permettent de lier l'angle du rayon incident avec ceux des rayons réfléchis et réfracté et déterminer les coefficients de réflexion et de transmission à la jonction entre 2 milieux, par exemple du plastique et de l'air. En connaissant l'angle d'incidence et l'impédance des matériaux en présence, laquelle est contenue dans des tables disponibles pour les principaux matériaux utilisés en mécanique, on peut donc déterminer l'intensité du rayon réfléchi ainsi que l'intensité et l'angle du rayon réfracté ce qui permet de définir les géométries souhaitées en fonction des différentes applications de l'invention.

Comme illustré schématiquement à la figure 3, le capteur 4 est constitué d'un support accueillant côte à côte un émetteur 5 et un récepteur 6, l'émetteur 5 et le récepteur 6 pouvant être la même pièce alors appelée émetteur-récepteur.

Dans une variante, le capteur peut aussi recevoir plusieurs émetteurs et ou plusieurs récepteurs selon les configurations envisagées.

De préférence le capteur est un élément qui peut être testé indépendamment de l'appareil qui doit le recevoir.

Un exemple d'application particulièrement avantageux est un dispositif de dialyse à domicile selon l'invention qui s'applique aux patients ayant une défaillance rénale chronique. La dialyse consiste à faire circuler, au moyen de pompes, le sang du patient dans un dialyseur pourvu d'une membrane semi-perméable. De l'autre côté de la membrane on fait circuler un liquide appelé « dialysat » qui va attirer à lui les impuretés contenues dans le sang du fait de la différence de concentration présente de part et d'autre de la membrane selon un procédé connu appelé « diffusion ». Ce traitement nécessite un appareil qui gère les débits de sang et de dialysat ainsi que la sécurité du patient, un dialyseur, des poches qui contiennent du dialysat frais et d'autres qui recueilleront le dialysat sorti du dialyseur. Ce traitement se déroule en général de 2 à 4 heures tous les jours ou tous les 2 jours selon la situation du patient. Dans certains cas il se fait de nuit, pendant environ 8 heures alors que le patient dort. Pour un tel dispositif il est connu que la boucle de circulation du sang doit être équipée d'un détecteur de présence d'air dans le sang avant que celui-ci ne soit réinjecté au patient.

Dans cet exemple de réalisation particulier, le dispositif de dialyse incorporant un dispositif selon l'invention comprend avantageusement au moins un détecteur constitué d'une cassette et d'un capteur tel que décrit précédemment.

## Revendications

1. Ensemble pour la détection de la présence d'un gaz dans un liquide confiné ou circulant dans une conduite comprenant un élément de support de la conduite (7) et un capteur (4) permettant l'émission et la réception d'onde acoustique ou lumineuse, le capteur (4) étant agencé sur le support (7) de la conduite en regard d'un seul et même côté de la conduite et l'élément de support comportant un guide d'onde (9) apte à acheminer l'onde émise par l'émetteur (5) du capteur (4) vers le récepteur (6) du capteur, **caractérisé en ce que** l'élément de support est constitué d'une cassette (7) et **en ce que** la conduite de liquide est formée par un évidement (10) réalisé dans le corps de la cassette et obturé par une membrane souple (8).

2. Ensemble selon la revendication 1, **caractérisé en ce que** la membrane (8) est fixée entre le capteur (4) et une partie rigide du support (7).

3. Ensemble selon l'une des revendications précédentes, **caractérisé en ce que** le guide d'onde (9) est constitué par des flancs agencés dans le corps du support (7).

4. Ensemble selon la revendication 3, **caractérisé en ce que** les flancs constituant le guide d'onde (9) forment un angle de 45 degrés avec le plan vertical.

5. Ensemble selon l'une des revendications précédentes, **caractérisé en ce que** ledit élément de support (7) de la conduite est réalisé dans une pièce moulée, injectée ou usinée dans un bloc de matière.

6. Ensemble selon l'une des revendications précédentes, **caractérisé en ce que** le capteur (4) est constitué d'un émetteur (5) et d'un récepteur (6) placés dans un même plan.

7. Ensemble selon l'une des revendications 1 à 5, **caractérisé en ce que** le capteur est constitué d'un émetteur-récepteur.

## Patentansprüche

1. Einheit zur Detektion des Vorhandenseins eines Gases in einer Flüssigkeit, die eingeschlossen ist oder in einer Leitung umläuft, die ein Element zum Stützen der Leitung (7) und einen Sensor (4) umfasst, der die Sendung und den Empfang von akustischen oder Lichtwellen ermöglicht, wobei der Sensor (4) auf der Stütze (7) der Leitung einer und derselben Seite der Leitung zugewandt eingerichtet ist und das Stützelement einen Wellenleiter (9) umfasst, der geeignet ist, die durch den Sender (5) des Sensors (4) gesendete Welle hin zu dem Empfänger (6) des Sensors zu befördern, **dadurch gekennzeichnet, dass** das Stützelement aus einer Kassette (7) besteht, und dadurch, dass die Flüssigkeitsleitung durch eine Aussparung (10) gebildet ist, die in dem Körper der Kassette ausgeführt ist und durch eine biegsame Membran (8) verschlossen ist.

2. Einheit nach Anspruch 1, **dadurch gekennzeichnet, dass** die Membran (8) zwischen dem Sensor (4) und einem starren Teil der Stütze (7) befestigt ist.

3. Einheit nach einem der vorhergehenden Ansprüche, **dadurch gekennzeichnet, dass** der Wellenleiter (9) aus Flanken besteht, die in dem Körper der Stütze (7) eingerichtet sind.

4. Einheit nach Anspruch 3, **dadurch gekennzeichnet, dass** die Flanken, die den Wellenleiter (9) bilden, einen Winkel von 45 Grad mit der vertikalen Ebene bilden.

5. Einheit nach einem der vorhergehenden Ansprüche, **dadurch gekennzeichnet, dass** das Stützelement (7) der Leitung aus einem geformten, gespritzten oder in einem Materialblock bearbeiteten Teil ausgeführt ist.

6. Einheit nach einem der vorhergehenden Ansprüche, **dadurch gekennzeichnet, dass** der Sensor (4) aus einem Sender (5) und einem Empfänger (6) besteht, die in einer gleichen Ebene platziert sind.

7. Einheit nach einem der Ansprüche 1 bis 5, **dadurch gekennzeichnet, dass** der Sensor aus einem Sendeempfänger besteht.

## Claims

1. Assembly for detecting the presence of a gas in a liquid contained or circulating in a conduit comprising an element (7) for supporting the conduit and a sensor (4) allowing the transmission and reception of an acoustic or light wave, the sensor (4) being arranged on the support (7) of the conduit facing the one and the same side of the conduit and the support element comprising a waveguide (9) capable of routing the wave transmitted by the transmitter (5) of the sensor (4) to the receiver (6) of the sensor, **characterised in that** the support element is formed by a cassette (7) and **in that** the liquid conduit is formed by a recess (10) produced in the body of the cassette and sealed by a flexible membrane (8).

2. Assembly as claimed in claim 1, **characterised in that** the membrane (8) is fixed between the sensor (4) and a rigid part of the support (7).

3. Assembly as claimed in any one of the preceding claims, **characterised in that** the waveguide (9) is formed by faces arranged in the body of the support (7).

4. Assembly as claimed in claim 3, **characterised in that** the faces forming the waveguide (9) form a 45° angle with the vertical plane.

5. Assembly as claimed in any one of the preceding claims, **characterised in that** said element (7) for supporting the conduit is produced in a part moulded, injected or machined in a block of material.

6. Assembly as claimed in any one of the preceding claims, **characterised in that** the sensor (4) is formed by a transmitter (5) and a receiver (6) placed in a same plane.

7. Assembly as claimed in any one of claims 1 to 5, **characterised in that** the sensor is formed by a transceiver.
